# EUROPEAN PATENT SPECIFICATION

(11) **EP 3 502 050 B1**
(45) Date of publication and mention of the grant of the patent: **21.12.2022**
(21) Application number: 16914142.1
(22) Date of filing: 22.08.2016
(51) Int. Cl.: C01B 15/027, A61L 2/18

(54) **METHOD FOR PRODUCING AQUEOUS HYDROGEN PEROXIDE**
VERFAHREN ZUR HERSTELLUNG VON WÄSSRIGEM WASSERSTOFFPEROXID
PROCÉDÉ DE PRODUCTION DE PEROXYDE D'HYDROGÈNE AQUEUX

(43) Date of publication of application: 26.06.2019
(73) Proprietor: Fuji Corporation, Chiryu-shi, Aichi 472-8686 (JP)
(72) Inventor: NIWA Tomoko, Chiryu, Aichi 472-8686 (JP); JINDO Takahiro, Chiryu, Aichi (JP); NIWA Akihiro, Chiryu Aichi (JP)
(74) Representative: Grünecker Patent- und Rechtsanwälte PartG mbB
(86) International application number: PCT/JP2016/074421
(87) International publication number: WO 2018/037467

(56) References cited:
- JP-A- 2002 316 041
- JP-A- 2012 204 248
- JP-A- 2013 128 909
- JP-A- 2014 189 441
- JP-A- 2015 085 297
- Hiroshi Kuwahata ET AL: "Generation of H2O2 in Distilled Water Irradiated with Atmospheric-Pressure Plasma Jet", e-Journal of Surface Science and Nanotechnology, 1 January 2013 (2013-01-01), pages 113-115, XP055657241, DOI: 10.1380/ejssnt.2013.113 Retrieved from the Internet: URL:https://www.jstage.jst.go.jp/article/e jssnt/11/0/11_113/_pdf

## Description

### Technical Field

The present invention relates to a hydrogen peroxide solution manufacturing method.

### Background Art

Because hydrogen peroxide solution has a disinfecting and sterilizing (hereinafter, disinfecting and sterilizing are collectively referred to as "disinfecting") effect, it is widely used for disinfecting medical equipment, food containers, and the like. As examples of hydrogen peroxide solution used as a disinfectant, it is known to process a syringe using hydrogen peroxide solution (refer to patent literature 1), and to process a cap of a PET bottle using hydrogen peroxide solution (refer to patent literature 2). Also, as a hydrogen peroxide solution manufacturing method, it is known to apply electromagnetic waves to pure water and to manufacture hydrogen peroxide solution from the resulting plasma (refer to patent literature 3).

### Citation List

### Patent Literature

Patent literature 1: JP-A-2014-28114
Patent literature 2: JP-A-2015-63324
Patent literature 3: JP-A-2014-189441

### Summary of Invention

### Technical Problem

When using hydrogen peroxide solution to disinfect medical equipment, food containers, and the like, as in patent literature 1 and 2, it is desirable not to leave a residue of hydrogen peroxide solution after processing. However, commercially sold hydrogen peroxide solution is mixed with a stabilizer such that the concentration of the hydrogen peroxide solution is fixed. Thus, as disclosed in patent literature 2, when there is a concern about residual hydrogen ·peroxide solution, it is necessary to adjust the amount of hydrogen peroxide solution introduced, or to apply steam.

Further, a hydrogen peroxide solution manufacturing method disclosed in patent literature 3 is useful for cases in which large quantities of hydrogen peroxide solution are required such as when cleaning boards in a semiconductor manufacturing process. However, when disinfecting medical equipment and the like, it is desirable to, in a specified location, manufacturer small quantities of hydrogen peroxide solution and to have the hydrogen peroxide decompose after disinfecting. Until now, there has not been a hydrogen peroxide solution manufacturing method that can use a small device to efficiently manufacture small quantities of hydrogen peroxide solution and to have the hydrogen peroxide decompose over time.

Hiroshi Kuwahata ET AL: "Generation of H2O2 in Distilled Water Irradiated with Atmospheric-Pressure Plasma Jet", Surface Science and Nanotechnology, 1 January 2013 (2013-01-01), pages 113-115, DOI: 10.1380/ejssnt.2013.113, discloses a process for generating hydrogen peroxide by irradiating an argon plasma jet into water.

To solve the above problems, an object of the present invention is to provide a hydrogen peroxide solution manufacturing method that that uses a small device to efficiently manufacture small quantities of hydrogen peroxide solution and has the hydrogen peroxide decompose over time.

### Solution to Problem

To solve the above problems, a hydrogen peroxide solution manufacturing method (hereinafter also referred to simply as "manufacturing method") of the present disclosure includes generating plasma by performing electrical discharge into an inert gas under an atmosphere purged using inert gas, and ejecting the generated plasma into water characterized in that for the generating of the plasma, the inert gas includes oxygen.

### Advantageous Effects

With a hydrogen peroxide solution manufacturing method of the present disclosure, by ejecting plasma generated by performing electrical discharge into an inert gas under an atmosphere purged using inert gas, it is possible to manufacture hydrogen peroxide solution with sufficient disinfecting power and that has the hydrogen peroxide solution decompose over time.

### Brief Description of Drawings

Fig. 1

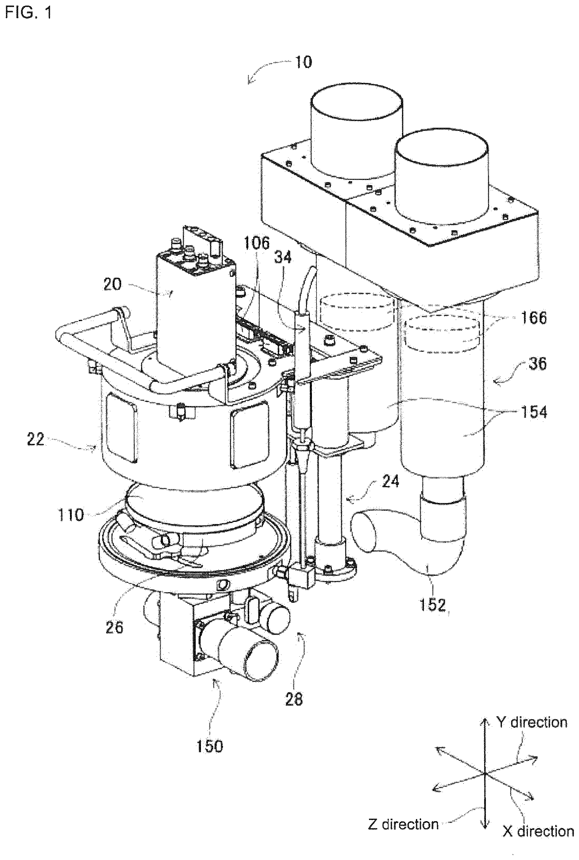

   Fig. 1 is a perspective view of a plasma emitting device.
Fig. 2

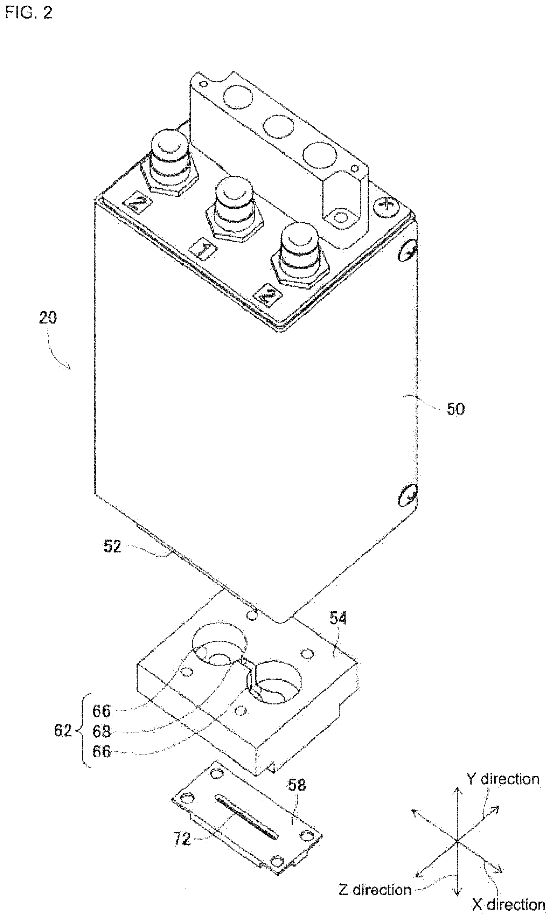

   Fig. 2 is an exploded view of a plasma generating device.
Fig. 3

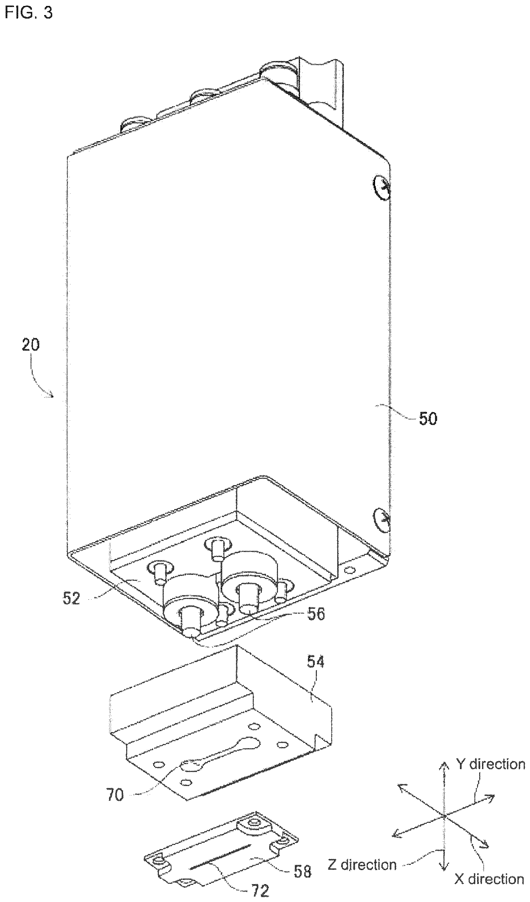

   Fig. 3 is an exploded view of the plasma generating device.
Fig. 4

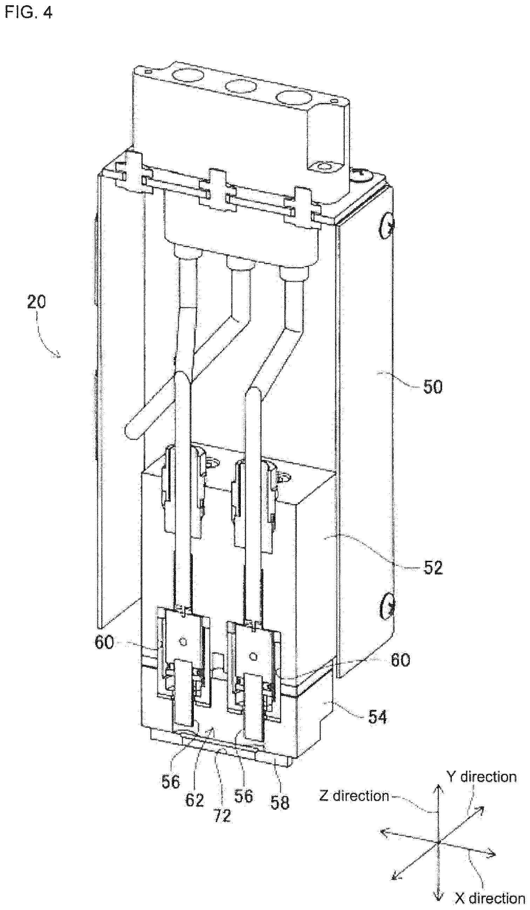

   Fig. 4 is a sectional view of the plasma generating device.
Fig. 5

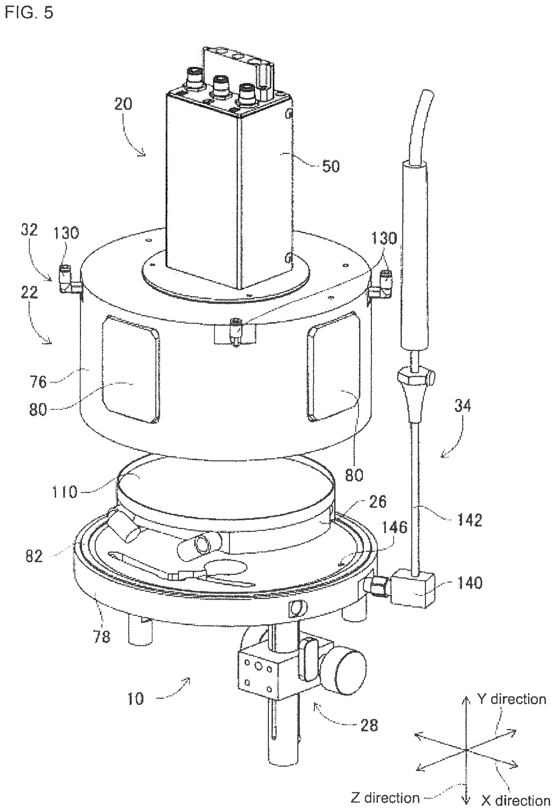

   Fig. 5 is a perspective view of the plasma emitting device.
Fig. 6

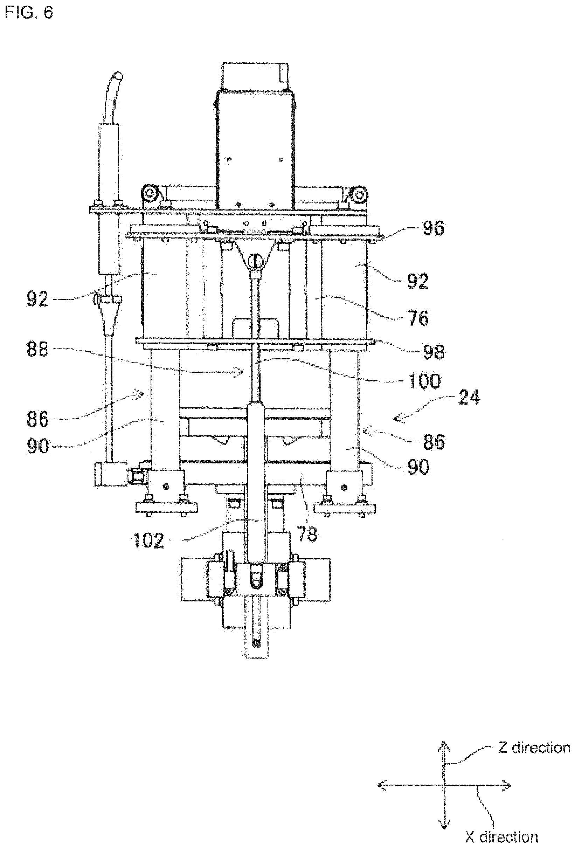

   Fig. 6 is a side view of the plasma emitting device.
Fig. 7

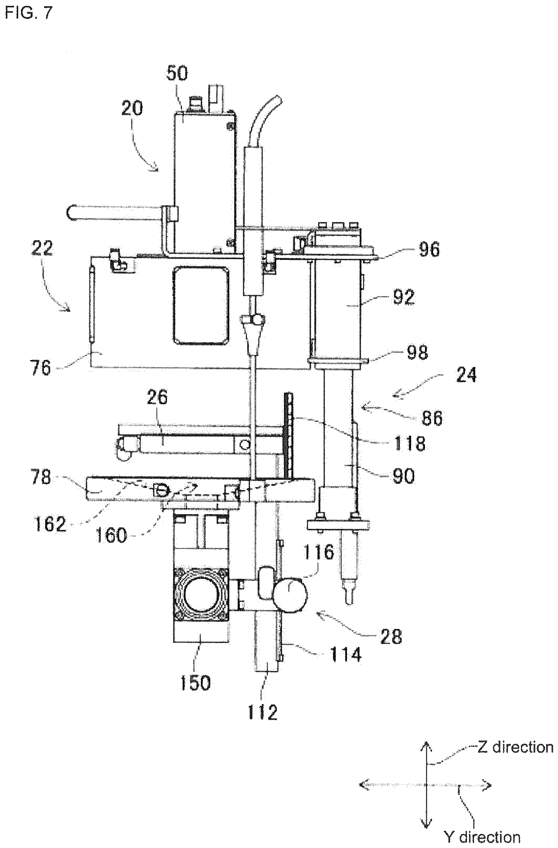

   Fig. 7 is a side view of the plasma emitting device.
Fig. 8

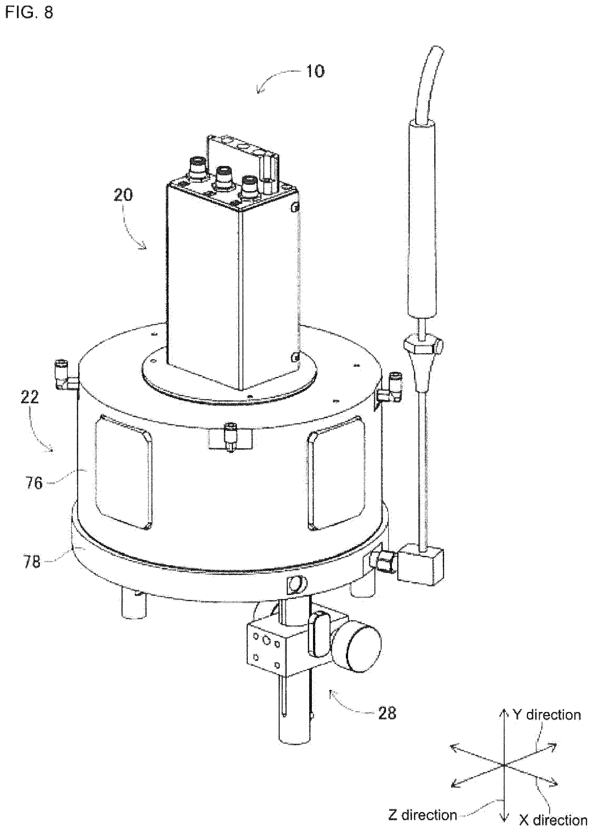

   Fig. 8 is a perspective view of the plasma emitting device.
Fig. 9

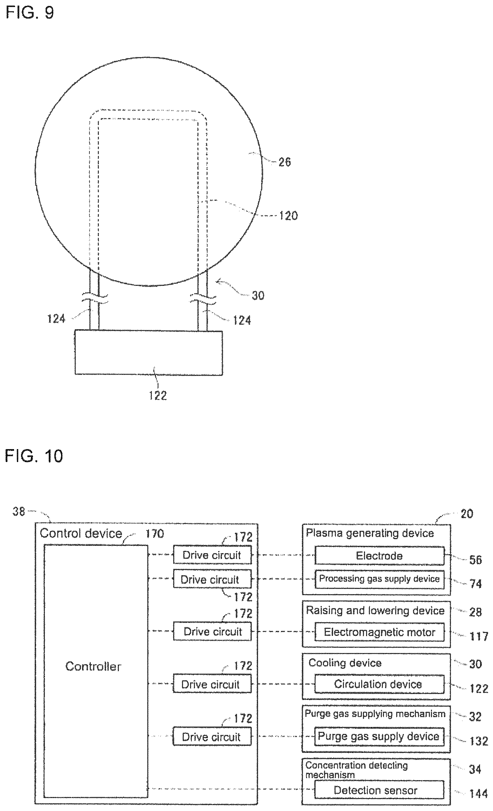

   Fig. 9 is a schematic diagram of a cooling device.
Fig. 10
   Fig. 10 is a block diagram of a control device.
Fig. 11

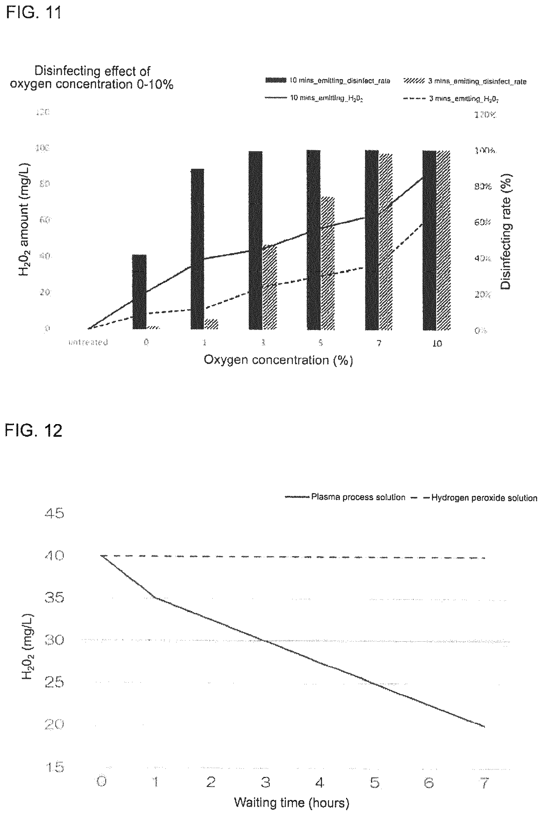

   Fig. 11 is a graph showing the amount of hydrogen peroxide solution and the disinfecting effect inside the hydrogen peroxide solution (medium) manufactured by the manufacturing method of the present disclosure.
Fig. 12
   Fig. 12 is a graph showing the decomposing over time of the amount of hydrogen peroxide solution inside the hydrogen peroxide solution (medium) manufactured by the manufacturing method of the present disclosure.
Fig. 13

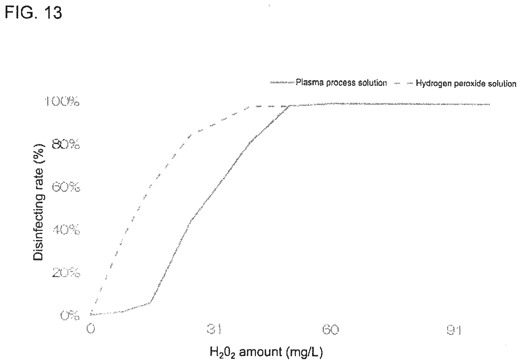

   Fig. 13 is a graph showing the disinfecting effect on common bacteria when using the hydrogen peroxide solution (medium) manufactured by the manufacturing method of the present disclosure and when using a commercially available hydrogen peroxide solution.

### Description of Preferred Embodiments

The following describes in detail referring to the figures an example embodiment of the present invention.

### Hydrogen peroxide solution manufacturing method

A manufacturing method of the present disclosure includes generating plasma by performing electrical discharge into an inert gas under an atmosphere purged using inert gas, and ejecting the generated plasma into water, wherein for the generating of the plasma, the inert gas includes oxygen.

Because plasma includes active radicals, when it reacts with oxygen, ozone is formed, decreasing the manufacturing efficiency of the hydrogen peroxide solution. Therefore, when ejecting the plasma into water, it is desirable to manage the atmosphere around the water by purging it with inert gas. The insert gas used is not particularly limited so long as it is a gas generally used in the field, such as argon or nitrogen. Also, the purge method may be any general method used in the field.

With a manufacturing method of the present disclosure, hydrogen peroxide solution is manufactured by ejecting plasma generated by performing electrical discharge in an inert gas under an atmosphere purged using inert gas, and ejecting the generated plasma into water, wherein for the generating of the plasma, the inert gas includes oxygen. Accordingly, it is possible to make a device smaller than a conventional manufacturing device as disclosed in patent literature 3 and the like, and a specified amount of hydrogen peroxide solution can be manufactured in a specified location. So long as the hydrogen peroxide solution is capable of disinfecting, there is no particular limit to the concentration of the hydrogen peroxide solution, but as given in the embodiment below, if the concentration is about 22.5 mg/L, it is possible to disinfect common bacteria that exist naturally in soil and the like. The concentration of the hydrogen peroxide solution manufactured may be controlled by adjusting a flow of the inert gas supplied to the plasma emitting device or the emitting time of the plasma into the water. Also, gas supplied to the plasma emitting device may be only inert gas, or may be inert gas mixed with oxygen. When oxygen is mixed with the inert gas, for the same flow amount of inert gas used for generating plasma supplied to the plasma emitting device, it is possible to manufacture a higher concentration of hydrogen peroxide solution, thus improving manufacturing efficiency. The concentration of oxygen in the inert gas is not particularly limited, so long as the concentration is within a range that improves the manufacturing efficiency of the hydrogen peroxide solution, but due to the fact that water will evaporate if the oxygen concentration is too high, it is necessary to adjust such that the temperature is 100 degrees Celsius or cooler.

Hydrogen peroxide solution manufactured using the manufacturing method of the present disclosure, unlike commercially available hydrogen peroxide solution, decomposes over time such that the concentration drops. Accordingly, it is useful for disinfecting disinfecting targets for which it is a problem if residual hydrogen peroxide solution remains. The disinfecting target is not particularly limited, but it is especially useful for items that will contact a human body, such as medical equipment, packaging for foodstuffs, beds, sheets, clothes, slippers, and the like. Disinfecting of a disinfecting target may be performed by applying the manufactured hydrogen peroxide solution to the disinfecting target, or dipping the disinfecting target into the manufactured hydrogen peroxide solution.

### Plasma emitting device used for the hydrogen peroxide solution manufacturing

With the manufacturing method of the present disclosure, hydrogen peroxide solution is manufactured by ejecting plasma into water under an atmosphere purged using inert gas, and ejecting the generated plasma into water, wherein for the generating of the plasma, the inert gas includes oxygen. Accordingly, the plasma emitting device is used to eject plasma towards water, and, as described in an embodiment below, oxygen is added to the inert gas, hence, the manufacturing efficiency of the hydrogen peroxide solution improves. Therefore, it is desirable for the plasma emitting device to be capable of adjusting the mixing ratio of inert gas and oxygen.

Fig. 1 shows an example of a plasma emitting device that can be used for a manufacturing method of the present disclosure. Plasma emitting device 10 is for emitting plasma to water under atmospheric pressure and is provided with: plasma generating device 20, cover housing 22, opening and closing mechanism 24, stage 26, raising and lowering device 28, cooling device 30 (refer to fig. 9), purge gas supplying mechanism 32 (refer to fig. 5), concentration detecting mechanism 34, exhaust mechanism 36, control device 38 (refer to fig. 10). Note that, the width direction of plasma emitting device 10 is taken as the X direction, the depth direction of plasma emitting device 10 is taken as the Y direction, and the direction perpendicular to the X direction and the Y direction, that is, the vertical direction, is taken as the Z direction.

As shown in figs. 2 to 4, plasma generating device 20 includes cover 50, upper block 52, lower block 54, pair of electrodes 56, and nozzle block 58. Cover 50 is a substantially cuboid tube with a lid, and upper block 52 is arranged inside cover 50. Upper block 52 is substantially cuboid and formed from ceramic material. Pair of round column recessed sections 60 are formed in a lower surface of upper block 52.

Lower block 54 is also substantially cuboid and formed from ceramic material. Recessed section 62 is formed on an upper surface of lower block 54, with recessed section 62 being configured from pair of round column recess sections 66, and connecting recessed sections 68 that are connected to the pair of round column recessed sections 66. Lower block is fixed to a lower surface of upper block 52 in a state protruding from a lower end of cover 50, and round column recessed sections 60 of upper block 52 are connected to round column recessed sections 66 of lower block 54. Round column recessed sections 60 and round column recessed sections 66 have approximately the same diameters. Also, slit 70 piercing a lower surface of lower block 54 is formed on the bottom surface of recessed section 62.

Each of the pair of electrodes 56 is arranged in a round column of space demarcated by round column recessed sections 60 of upper block 52 and round column recessed sections 66 of lower block 54. The outer diameter of electrode 56 is smaller than the inner diameter of round column recessed sections 60 and 66. Nozzle block 58 is a substantially flat plate and is fixed to a lower surface of lower block 54. Emission port 58 is formed in nozzle block 58 connected to slit 70 of lower block 54 and piercing nozzle block 58 in the vertical direction.

Plasma generating device 20 further includes processing gas supply device 74 (refer to fig. 10). Processing gas supply device 74 supplies processing gas in which an active gas such as oxygen is mixed with an inert gas such as nitrogen at a given ratio, and is connected to a round column of space demarcated by round column recessed sections 60 and 66 and to an upper section of connecting recessed section 68, via piping (not shown). Thus, processing gas is supplied into recessed section 62 from an upper section of connecting recessed sections 68 and the gap between electrodes 56 and round column recessed sections 66.

According to such a configuration, plasma generating device 20 emits plasma from emission port 72 of nozzle block 58. In detail, processing gas is supplied into recessed section 62 from processing gas supply device 74. Here, in recessed section 62, a voltage is applied to the pair of electrodes 56 such that a current flows between the pair of electrodes. Thus, electrical discharge occurs between the pair of electrodes 56 resulting in plasmarizing of the processing gas. The plasma is then emitted from emission port 72 via slit 70.

As shown in fig. 5, cover housing 22 includes upper cover 76 and lower cover 78. Upper cover 76 is a substantially cylindrical tube with a lid, with a through-hole (not shown) formed corresponding to lower block 54 on plasma generating device 20 being formed in the lid of upper cover 76. Cover 50 of plasma generating device 20 is fixed to the lid of upper cover 76 in an upright state so as to cover the through-hole. Therefore, lower block 54 and nozzle block 54 of plasma generating device 20 protrude in the Z direction towards the inside of upper cover 76. Accordingly, plasma generated by plasma generating device 20 is emitted in the Z direction from emission port 72 of nozzle block 58 towards the inside of upper cover 76.

Further, substantially rectangular through-holes (not shown) are formed at three equidistant positions in a side surface of upper cover 76 and transparent glass plate 80 is arranged to cover those through-holes. Thus, it is possible to check inside upper cover 76 through glass plate 80.

Lower cover 78 of cover housing 22 is a substantially round plate and is fixed to the housing (not shown) of a mounting section on which plasma emitting device 10 is mounted. The outer diameter of lower cover 78 is greater than the outer diameter of upper cover 76 and ring-shaped packing 82 of the same diameter as upper cover 76 is installed on an upper surface of lower cover 78. Further, by upper cover 76 being slid downwards by opening and closing mechanism 24, upper cover 76 closely contacts packing 82, thereby sealing the inside of cover housing 22.

In detail, as shown in figs. 6 and 7, opening and closing mechanism 24 includes pair of slide mechanisms 86 and air cylinder 88. Each slide mechanism 86 includes support shaft 90 and slider 92. Support shaft 90 is provided upright extending in the Z direction of the housing of the mounting section. Slide 92 is a substantially cylindrical tube, and is engaged with support shaft 90 so as to be slidable in an axial direction of support shaft 90. Upper cover 76 is held on slider 92 by lower bracket 96 and upper bracket 98. Accordingly, upper cover 76 is able to slide in the Z direction, that is, the vertical direction.

Air cylinder 88 includes rod 100, a piston (not shown), and cylinder 102. Rod 100 is arranged extending in the Z direction, and is fixed at an upper end to upper cover 76. The piston is fixed to a lower end of rod 100. The piston fits inside cylinder 102 from above and is slidable inside cylinder 102. Cylinder 102 is fixed at a lower end to the housing of the mounting section, and a specified quantity of air is sealed inside cylinder 102.

Thus, air cylinder 88 functions as a damper and sudden falling of upper cover 76 is prevented. The air pressure inside cylinder 102 is a pressure that can be compressed by the weight of items that slide with upper cover 76, that is, upper cover 76, plasma generating device 20, slider 92, and the like. In other words, when an operator releases upper cover 76 in a state with upper cover 76 lifted, upper cover 76 is lowered by the weight of upper cover 76 itself and the like. As shown in fig. 8, upper cover 76 contacts closely with packing 82 lower cover 78 such that the inside of cover housing 22 is sealed by upper cover 76 and lower cover 78.

The inside of cover housing 22 is opened by an operator raising upper cover 76. Magnet 106 (refer to fig. 1) is fixed to an upper surface of upper cover 76 and by upper cover 76 being raised, magnet 106 sticks to the housing of the mounting section. In this manner, by magnet 106 sticking to the housing of the mounting section, upper cover 76 is maintained in a raised state, that is, cover housing 22 is maintained in an open state.

Stage 26 is substantially a round plate and dish 110 is loaded on an upper surface of stage 26. The outer diameter of stage 26 is smaller than the outer diameter of lower cover 78. Also, stage 26 is arranged on an upper surface of lower cover 78.

As shown in fig. 7, raising and lowering device 28 includes support rod 112, rack 114, pinion 116, and electromagnetic motor 117 (refer to fig. 10). A through-hole (not illustrated) which pierces lower cover 78 in the up-down direction is formed in lower cover 78 and support rod 112 is inserted through the through-hole. The outer diameter of support rod 112 is smaller than the inner diameter of the through-hole and support rod 112 is capable of moving in the up-down direction, that is, the Z direction. A lower surface of stage 26 is fixed to the upper end of support rod 112.

Rack 114 is fixed to an outer circumferential surface of a portion of support rod 112 that extends downwards from lower cover 78 such that rack 114 extends in the axial direction of support rod 112. Pinion 116 engages with rack 114 and rotates through the driving of electromagnetic motor 117. Pinion 116 is held by the housing of the mounting section to be capable of rotating. According to this structure, support rod 112 moves in the Z direction and stage 26 is lifted and lowered due to pinion 116 rotating via the driving of electromagnetic motor 117. Measurement rod 118 is provided upright next to stage 26 on the upper surface of lower cover 78. A scale is provided on an outer circumferential surface of measurement rod 118, the scale being used to check the height in the Z direction of stage 26, that is, the lifting and lowering amount of stage 26 can be checked visually using the scale.

As shown in fig. 9, cooling device 30 includes cooling water path 120, circulation device 122, and pipe 124. Cooling water path 120 penetrates an inner portion of stage 26 along the surface direction in a C-shape and opens at two locations on the outer circumferential surface of stage 26. Circulation device 122 is a device that cools water and circulates the cooled water. Circulation device 122 is connected to cooling water path 120 via pipe 124. Accordingly, stage 26 is cooled due to the cooling water flowing inside stage 26.

As shown in fig. 5, purge gas supplying mechanism 32 includes four air joints 130 (in the figure, three are illustrated) and purge gas supply device 132 (refer to fig. 10). The four air joints 130 are provided at equidistant positions on an upper end portion of a side surface of upper cover 76 and each of the air joints 130 is open to an inner portion of upper cover 76. Purge gas supply device 132 is a device that supplies an inert gas such as nitrogen and is connected to air joints 130 via a pipe (not illustrated). According to this structure, purge gas supplying mechanism 32 supplies an inert gas inside upper cover 76.

Concentration detecting mechanism 34 includes air joint 140, pipe 142, and detection sensor 144 (refer to fig. 10) A through-hole (not shown) that connects the upper face to the side surface of lower cover 78 is formed in lower cover 78. Opening 146 of the through-hole on the upper surface side of lower cover 78 is positioned on the inside of packing 82. On the other hand, air joint 140 is connected to the opening of the through-hole on the side surface side of lower cover 78. Detection sensor 144 is for detecting oxygen concentration and is connected to air joint 140 via pipe 142. According to this structure, concentration detecting mechanism 34 detects the oxygen concentration inside cover housing 22 when cover housing 22 is sealed.

As shown in fig. 1, exhaust mechanism 36 includes L-shaped pipe 150, connecting pipe 152, and main pipe 154. As shown in fig. 7, duct port 160 that opens to an upper surface and a lower surface of lower cover 78 is formed in the lower cover 78. The opening of duct port 160 on the upper surface side of lower cover 78 is tapered surface 162, the inner diameter of which increases towards the top. In other words, when cover housing 22 is sealed, the tapered surface 162 is inclined towards an inner wall surface of upper cover 76. Further, L-shaped pipe 150 is connected to the opening of duct port 160 on the lower surface side of lower cover 78 of duct port 160. Main pipe 154 is connected to L-shaped pipe 150 via connecting pipe 152. Note that, the portion of connecting pipe 152 on L-shaped pipe 150 is omitted. Ozone filter 166 is installed inside main pipe 154. Ozone filter 166 is formed of activated carbon and adsorbs ozone.

As shown in fig. 10, control device manufacturing 8 is provided with controller 170 and multiple drive circuits 172. The multiple drive circuits 172 are connected to electrodes 56, processing gas supply device 74, electromagnetic motor 117, circulation device 122, and purge gas supply device 132. Controller 170 is provided with a CPU, ROM, RAM, and so on, is formed mainly from a computer, and is connected to the multiple drive circuits 172. Thus, operations of plasma generating device 20, raising and lowering device 28, cooling device 30, and purge gas supply mechanism 32 are controlled by controller 170. Controller 170 is also connected to detection sensor 144. Thus, controller 170 acquires a detection result of detection sensor 144, that is, the oxygen concentration inside cover housing 22.

### Manufacturing of hydrogen peroxide solution using the above plasma emitting device

A hydrogen peroxide solution manufacturing method using the above plasma emitting device is described below. First, dish 110 filled with water is loaded on stage 26. Next, stage 26 is raised to a specified height by raising and lowering device 28. Thus, it is possible to set the distance between plasma emission port 72 and the water used as the plasma application target to a given distance. Note that, the raising and lowering height of stage 26 may be checked using the scale provided on measurement rod 118.

Next, upper cover 76 is lowered to seal cover housing 22. Then, inert gas is supplied inside cover housing 22 from purge gas supply mechanism 32. Here, the oxygen concentration inside cover housing 22 is detected by concentration detecting mechanism 34. Then, when the detected oxygen concentration is equal to or lower than a predetermined threshold value, plasma is emitted inside cover housing 22 by plasma generating device 20. Note that, inert gas continues to be supplied into cover housing 22 while plasma is being emitted.

In this manner, by supplying inert gas inside cover housing 22, the air inside cover housing 22 is expelled outside cover housing 22. Here, conditions that influence the plasma emissions are managed by adjusting the oxygen concentration inside cover housing 22. In detail, because plasma includes active radicals, when these react with oxygen, ozone is formed, reducing the effectiveness of the plasma emitting. Therefore, by adjusting the oxygen concentration inside cover housing 22, it is possible to investigate the influence that the oxygen concentration has with respect to the effect of water to which the plasma was omitted. Also, it is possible to emit plasma to water under the same conditions. By this, it is possible to manufacture hydrogen peroxide solution with the same quality.

Note that, during emission of plasma, cooling water is circulated inside stage 26 by cooling device 30. Accordingly, because stage 26 is cooled, an increase in the temperature of the water inside dish 110 caused by plasma being emitted is curtailed, making it possible to prevent evaporation of the water. Also, by managing the temperature of the cooling water, it is possible to adjust the temperature of the water during emission of plasma.

Also, duct port 160 is formed in lower cover 78. Therefore, by inert gas being supplied inside cover housing 22, the pressure inside cover housing 22 becomes positive, naturally ventilating inside cover housing 22. Further, tapered surface 162 with an inner diameter that increases in size towards an upper surface of lower cover 78 is formed in duct port 160 of lower cover 78. By this, the exhausting of gas from inside cover housing 22 is encouraged. Also, ozone filter 166 is provided on exhaust mechanism 36. By this, even in a case in which the plasma reacts with the oxygen and ozone is generated, it is possible to prevent the escape of ozone to the outside.

Further, because glass plate 80 through which it is possible to visually check inside upper cover 76 is provided in upper cover 76, it is possible to check the state of the emission of plasma.

Note that, plasma emitting device 10 is an example of a plasma emitting device. Plasma generating device 20 is an example of a plasma generating device. Cover housing 22 is an example of a cover housing. Stage 26 is an example of a stage. Raising and lowering device 28 is an example of a moving device. Cooling device 30 is an example of a cooling device. Control device 38 is an example of a control device. Emission port 72 is an example of an emission port. Purge gas supply device 132 is an example of a gas supply device. Detection sensor 144 is an example of a detection sensor. Duct port 160 is an example of a duct port. Tapered surface 162 is an example of a tapered surface. Also, detection sensor 144 is a sensor for detecting oxygen concentration, but a sensor for detecting another specified gas may be used.

Example embodiments of the present invention are described below and are presented simply to describe the present invention by reference to the specific forms of the embodiments. The specific forms of the present disclosure disclosed below are for describing the invention only, and do not limit or restrict the range of the invention as disclosed herein.

### Embodiments

### First embodiment

### Hydrogen peroxide solution (medium) manufacturing

Hydrogen peroxide solution (medium) is manufactured by the following procedure using the above plasma emitting device 10.
1. 10 mL of Dulbecco's Modified Eagle's Medium (made by Sigma-ALdrich, D5796) is put into a dish and the dish is set on plasma emitting device 10. To check the disinfecting effect, instead of pure water a medium is used for manufacturing the hydrogen peroxide solution.
2. After setting the dish on stage 26 of plasma emitting device 10, cover housing 22 is sealed.
3. After purging with Ar gas (10 L) (at this point, the oxygen concentration inside cover housing 22 is 0.1% or lower), plasma is emitted to the medium by applying voltage to the electrodes. Plasma emission is performed under the following conditions: gas flow supply of 2 L/min; distance between medium and plasma emission port of 4 mm; plasma emission time of 3 min or 10 min. During plasma emission, a mixture of Ar and oxygen is used for the gas, with the oxygen concentration of the mixed gas adjusted to be 0 to 15%. The amount of hydrogen peroxide of the manufactured hydrogen peroxide solution is measured using an automatic titrator (Hydrogen Peroxide Counter-HP-300, made by Hirunama Sangyo). Note that, because the hydrogen peroxide in the hydrogen peroxide solution (medium) manufactured by a manufacturing method of the present disclosure decomposes over time, measurement of the amount of hydrogen peroxide is performed quickly after manufacturing.

### Second embodiment

### Checking disinfecting effects of hydrogen peroxide solution (medium)

The disinfecting effect using the hydrogen peroxide solution (medium) manufactured in the first embodiment was checked by the following procedure.
1. By rinsing a commercially available vegetable partially covered in soil with a saline solution, common bacteria from the surface of a vegetable was collected in the saline solution. To check the disinfecting effect with respect to everyday common bacteria, without targeting a specific type of bacteria, bacteria on the surface of a commercially available vegetable were used.
2. After stirring 1 mL of saline solution and 1 mL of hydrogen peroxide solution (medium) manufactured in the first embodiment, they were introduced to a general bacteria detection medium (Sheet Medium Sanitakun, made by JNC).
3. The bacteria count was checked after 24 hours cultivation at 36 degrees Celsius.

Table 1 and fig. 11 show the disinfecting effect and concentration of hydrogen peroxide in the hydrogen peroxide solution (medium) manufactured in the first embodiment. The concentration of the hydrogen peroxide solution (medium) given in table 1 and fig. 11 is the concentration of the hydrogen peroxide solution (medium) manufactured in the first embodiment. As in the second procedure above, when checking the disinfecting effect, saline solution of an amount the same as the hydrogen peroxide solution (medium) was added. Accordingly, the concentration of the hydrogen peroxide solution (medium) when the disinfecting effect was actually checked was half the concentration of the hydrogen peroxide solution (medium) given in table 1 and fig. 1.

**Table 1**

| | | Oxygen concentration (%) | | | | | | | |
|---|---|---|---|---|---|---|---|---|---|
| | | Unprocessed | 0 | 1 | 3 | 5 | 7 | 10 | 15 |
| Emission for 10 minutes | H₂O₂ amount (mg/L) | 0 | 20.69 | 39.21 | 44.78 | 56.36 | 64.37 | 91.15 | 132.8 |
| | Disinfectant rate (%) | 0% | 41% | 89% | 99% | 100% | 100% | 100% | - |
| Emission for 3 minutes | H₂O₂ amount (mg/L) | 0 | 8.78 | 11.69 | 23.82 | 29.99 | 36.2 | 66.47 | 87.44 |
| | Disinfectant rate (%) | 0% | 2% | 6% | 47% | 74% | 98% | 100% | - |

As shown in fig. 11 and table 1, as the oxygen concentration in the mixed gas increases, and as the time over which the plasma is emitted increases, highly concentrated hydrogen peroxide solution (medium) is manufactured. Also, when the amount of hydrogen peroxide in the hydrogen peroxide solution (medium) shown in fig. 11 and table 1 is 44.78 mg/L, a disinfecting effect of approximately 99% is seen. Accordingly, for everyday common bacteria, approximately 22.5 mg/L of hydrogen peroxide solution clearly gives a sufficient disinfecting effect.

### Third embodiment

### Hydrogen peroxide solution (medium) manufacturing with changed gas flow amount

Hydrogen peroxide solution (medium) was manufactured and the hydrogen peroxide amount was measured using the same procedure as the first embodiment, except that the gas flow amount during plasma emission and the oxygen concentration were that shown in table 2. The measured hydrogen peroxide amount is also shown in table 2.

**Table 2**

| Hydrogen peroxide solution amount (mg/L) | | | | | | |
|---|---|---|---|---|---|---|
| | | Oxygen concentration (%) | | | | |
| | | 0 | 0.5 | 1 | 3 | 5 |
| Gas flow (L/min) | 1 | 8.61 | 13.93 | 22.28 | 31.73 | 34.81 |
| | 2 | 20.69 | 26 | 39.21 | 44.78 | 56.36 |
| | 3 | 128.3 | 134.8 | 140.1 | 141.9 | 149.1 |
| | 4 | 171.7 | 196.9 | 220.4 | 224.8 | 228.2 |

As shown in table 2, as the gas flow amount increases, and as the oxygen concentration in the gas increases, the amount of hydrogen peroxide in the manufactured hydrogen peroxide solution (medium) increases.

### Fourth embodiment

### Change over time of the amount of hydrogen peroxide

The change over time of the amount of hydrogen peroxide for the hydrogen peroxide solution (medium) with an amount of hydrogen peroxide of approximately 40 mg/L manufactured using the same procedure as the first embodiment and for a commercially available hydrogen peroxide solution diluted to approximately 40 mg/L (Oxydol, made by Kenei Pharmaceutica, with concentration adjusted by being diluted in DMEM medium) was measured. Fig. 12 is a graph showing the change over time of the amount of hydrogen peroxide. As shown in fig. 12, the commercially available hydrogen peroxide solution showed no change in the amount of hydrogen peroxide over time. On the other hand, with the hydrogen peroxide solution manufactured with a manufacturing method of the present disclosure, the hydrogen peroxide decomposed, such that the amount of hydrogen peroxide approximately halved in seven hours. The above results confirm that because the hydrogen peroxide solution manufactured with a manufacturing method of the present disclosure decomposes over time, it is useful for disinfecting disinfecting targets for which there is a concern about residual hydrogen peroxide.

### Fifth embodiment

### Disinfecting effects compared to commercially available hydrogen peroxide solution

The commercially available hydrogen peroxide solution was diluted to various concentrations and the disinfecting effects were checked using the same procedure as the second embodiment. Fig. 13 is a graph showing the disinfecting effect on common bacteria when using the hydrogen peroxide solution (medium) manufactured by the manufacturing method of the present disclosure and when using a commercially available hydrogen peroxide solution. The concentration of the hydrogen peroxide solution (medium) in fig. 13 is the concentration before adding the saline solution, and the concentration of the hydrogen peroxide solution (medium) for which the disinfecting effects where actually checked is half the concentration of the hydrogen peroxide solution (medium) shown in fig. 13. As shown in fig. 13, with the low concentration, the disinfecting effect of the commercially available hydrogen peroxide solution was higher than that of the hydrogen peroxide solution manufactured by a manufacturing method of the present disclosure. However, there was not a great difference in the concentration for which the disinfecting rate become just about 100%, and with hydrogen peroxide solution with a concentration of approximately 22.5 mg/L (45 mg/L in fig. 13) and higher, disinfecting effects of 100% were seen. This clearly shows that hydrogen peroxide solution manufactured by a manufacturing method of the present disclosure has sufficient disinfecting effects.

### Reference Signs List

10: plasma emitting device;
20: plasma generating device;
22: cover housing;
26: stage;
28: raising and lowering device (moving device);
30: cooling device;
38: control device;
72: emission port;
132: purge gas supply device (gas supply device);
144: detection sensor;
160: duct outlet;
162: tapered surface

## Claims

1. A hydrogen peroxide solution manufacturing method comprising:
generating plasma by performing electrical discharge into an inert gas under an atmosphere purged using inert gas, and
ejecting the generated plasma into water
**characterized in that:**
for the generating of the plasma, the inert gas includes oxygen.

2. The hydrogen peroxide solution manufacturing method according to claim 1, wherein
by adjusting a flow of the inert gas supplied to the water or the emitting time of the plasma into the water, in the ejecting of the generated plasma into the water, an amount of hydrogen peroxide in the obtained hydrogen peroxide solution is at least 22.5 mg/L.

3. The hydrogen peroxide solution manufacturing method according to claim 1 or 2, wherein
the amount of hydrogen peroxide in the hydrogen peroxide solution manufactured by the manufacturing method halves in seven hours when a hydrogen peroxide solution with an amount of hydrogen peroxide of approximately 40 mg/L is manufactured.

## Patentansprüche

1. Ein Verfahren zur Herstellung von Wasserstoffperoxidlösung, umfassend:
Erzeugung eines Plasmas durch elektrische Entladung in einem Inertgas unter einer mit Inertgas gespülten Atmosphäre und
Ausstoßen des erzeugten Plasmas in Wasser
**dadurch gekennzeichnet dass:**
für die Erzeugung des Plasmas, das Inertgas Sauerstoff enthält.

2. Das Verfahren zur Herstellung von Wasserstoffperoxidlösung gemäß Anspruch 1, wobei
durch Einstellen einer Strömung des dem Wasser zugeführten Inertgases oder der Emissionszeit des Plasmas in das Wasser beim Ausstoßen des erzeugten Plasmas in das Wasser eine Menge an Wasserstoffperoxid in der erhaltenen Wasserstoffperoxidlösung mindestens 22,5 mg/L ist.

3. Das Verfahren zur Herstellung von Wasserstoffperoxidlösung gemäß Anspruch 1 oder 2, wobei
die Menge an Wasserstoffperoxid in der durch das Herstellungsverfahren hergestellten Wasserstoffperoxidlösung sich in sieben Stunden halbiert, wenn eine Wasserstoffperoxidlösung mit einer Wasserstoffperoxidmenge von etwa 40 mg/L hergestellt wird.

## Revendications

1. Procédé de fabrication d'une solution de peroxyde d'hydrogène comprenant :
la génération d'un plasma par la réalisation d'une décharge électrique dans un gaz inerte sous une atmosphère purgée au moyen d'un gaz inerte, et
l'éjection du plasma généré dans de l'eau
**caractérisé en ce que**
pour la génération du plasma, le gaz inerte contient de l'oxygène.

2. Procédé de fabrication d'une solution de peroxyde d'hydrogène selon la revendication 1, dans lequel
en réglant un débit du gaz inerte fourni à l'eau ou la durée d'émission du plasma dans l'eau, pendant l'éjection du plasma généré dans l'eau, une quantité de peroxyde d'hydrogène dans la solution de peroxyde d'hydrogène obtenue est au moins égale à 22,5 mg/L.

3. Procédé de fabrication d'une solution de peroxyde d'hydrogène selon la revendication 1 ou 2, dans lequel
la quantité de peroxyde d'hydrogène dans la solution de peroxyde d'hydrogène fabriquée par le procédé de fabrication est réduite de moitié en sept heures quand on fabrique une solution de peroxyde d'hydrogène ayant une quantité de peroxyde d'hydrogène d'environ 40 mg/L.
